Europäisches Patentamt

European Patent Office

Office européen des brevets

(11) Publication number: **0 273 266**
**A2**

# EUROPEAN PATENT APPLICATION

(21) Application number: 87118308.3

(22) Date of filing: 10.12.87

(51) Int. Cl.⁴ **A61K 31/695**

(30) Priority: 17.12.86 US 942891

(43) Date of publication of application:
06.07.88 Bulletin 88/27

(84) Designated Contracting States:
DE FR GB

(71) Applicant: DOW CORNING CORPORATION
P.O. Box 1767
Midland Michigan 48640(US)

(72) Inventor: Ward, Andrew Hamilton
564 W. Birch Road
Sanford Michigan(US)

(74) Representative: Sternagel, Hans-Günther, Dr.
et al
Patentanwälte Dr. Michael Hann Dr. H.-G.
Sternagel Sander Aue 30
D-5060 Bergisch Gladbach 2(DE)

(54) Medicament containing a silicon derivative of menthol.

(57) Topically applied medicaments are described which contain a menthol derivative of the formula
$$R_{4-x}Si(OC_{10}H_{19})_x$$
wherein the group $OC_{10}H_{19}$ represents the menthoxy radical; R denotes an alkyl radical, alkoxy radical, a phenyl radical, or a phenoxy radical; and x has the value of 2, 3, or 4. The medicament provides sustained antipruritic and analgesic effects after skin treatment.

EP 0 273 266 A2

## MEDICAMENT CONTAINING A SILICON DERIVATIVE OF MENTHOL

This invention relates to a topically applied medicament which contains a silicon derivative of menthol as an active ingredient. The silicon derivative of menthol slowly hydrolyzes upon contact with water to release menthol, The slow release of menthol provides sustained antipruritic and analgesic effects after skin treatment.

The use of menthol in medical ointments and lotions to provide antipruritic and analgesic effects to reduce the symptoms of colds, sore or fatigued muscles, irritated or itchy skin is well known. For example, menthol is recognized as providing a counterirritant type of analgesic action when applied to the skin surface. A counterirritant analgesic replaces pain with another sensation which temporarily blocks the perception of the original pain. In the case of menthol, the replacement sensation is typically described as a "cooling" sensation.

Silicate ester derivatives of menthol are known materials which have been described, for example, by Allen et al., U.S. Patent Nos. 3,215,719 and 3,271,305. Allen et al. specifically describe the preparation of dimenthoxydiethoxysilane, $(C_{10}H_{19}O)_2Si(OC_2H_5)_2$. More generally, Allen et al. teach that silicate ester derivatives of fragrance alcohols can be used to treat textiles to provide a fragrance to the textiles that will last through several wash cycles.

Similarly, Cooper, U.K. Patent Application No. 2,042,890, teaches silicon derivatives of fragrance alcohols for use as fragrance-imparting components in antiperspirants and deodorant formulations. Cooper states that, at least in some cases, the silicon derivatives provide a longer lasting fragrance than the unmodified alcohols. Still other silicon derivatives of fragrance alcohols are disclosed by Yemoto et al., U.S. Patent No. 4,500,725, for use in liquid or solid articles for providing more persistent aromas.

While silicate esters of a number of primary alcohols have been found to hydrolyze at a rate sufficient to provide a detectable odor over extended periods of time, the corresponding esters of secondary alcohols generally hydrolyze so slowly under the use conditions that little if any odor is detectable. Consequently, silicate esters of secondary alcohols such as menthol have not proven useful as fragrance components.

The present invention relates to a medicament comprising (a) from 0.5 to 30 parts by weight of menthol derivative represented by the formula

$$R_{4-x}Si(OC_{10}H_{19})_x$$

wherein the group $OC_{10}H_{19}$ represents the menthoxy radical: R denotes a radical having 1 to 6 carbon atoms selected from the group consisting of alkyl radicals, alkoxy radicals, the phenyl radical, and the phenoxy radical; and x has the value of 2, 3, or 4 and (b) 70 to 100 parts by weight of a nonaqueous, physiologically acceptable carrier. The invention also relates to a method of treating skin to elicit an analgesic or antipruritic response comprising contacting skin with the medicament defined above.

The present invention relates to the use of certain silicon derivative of menthol as active ingredients in topically applied medicaments. The silicon derivatives of menthol which are useful in medicaments are described by the general formula

$$R_{4-x}Si(OC_{10}H_{19})_x \qquad I$$

wherein the group $OC_{10}H_{19}$ represents the menthoxy radical. The menthoxy radical should be understood to mean the residue remaining after removal of the hydrogen atom from the hydroxyl group of menthol.

In formula I, R denotes a radical having 1 to 6 carbon atoms selected from the group consisting of alkyl radicals, alkoxy radicals, the phenyl radical, and the phenoxy radical. The alkyl and alkoxy radicals may be branched or linear with linear radicals being preferred. For example, R may denote an alkyl radical such as methyl, ethyl, propyl, hexyl, isopropyl, isobutyl, neopentyl, and isohexyl or an alkoxy radical such as methoxy, ethoxy, propoxy, butoxy, isobutoxy, hexoxy, and isohexoxy. Generally, derivatives of formula I are preferred where R denotes methyl, ethyl, ethoxy, or propoxy because the precursor silanes for forming these derivatives are readily available and present minimal handling difficulties. When there are two R groups on the silicon atom, the R groups may be the same or different.

The menthol derivatives used in this invention have at least two menthoxy groups bonded to the silicon atom. This means that in formula I, x has a value of 2, 3, or 4. Only these derivatives having at least two menthoxy groups per silicon atom have been found effective to provide a prolonged analgesic effect when used in the medicament compositions of this invention. It should be understood, however, that derivatives with one menthoxy group per silicon atom may be formed as by-product during the preparation of the higher derivatives. While it is believed that such by-products are essentially ineffective in regard to generating the medicinal effects provided by the compositions of this invention, it is generally not necessary to separate the by-products from the active derivatives to prepare medicaments. Consequently, medicaments containing such by-products are within the scope of the present invention provided they contain sufficient quantity of the derivatives having at least two menthoxy groups.

The precise way in which an analgesic response is generated is not completely understood, but it is believed that the menthol derivatives hydrolyze upon sufficient contact with water to release menthol. The released menthol is then free to stimulate an analgesic response. It is believed that menthol derivatives with only one menthoxy group per silicon hydrolyze too slowly under the conditions in which the medicaments of this invention are used and thus do not generate a sufficient concentration of menthol to provide a useful analgesic response. The above theory is offered only as a possible explanation and is not intended to further limit or define the present invention.

Specific silicon derivatives of menthol which can be used in the medicaments of this invention include, for example, dimenthoxydimethoxysilane, dimenthoxydiethoxysilane, trimenthoxyethoxysilane, trimenthoxypropoxysilane, tetramenthoxysilane, methyltrimenthoxysilane, dipropyldimenthoxysilane, dimethyldimenthoxysilane, hexylmethyldimenthoxysilane, methyldimenthoxymethoxysilane, methyldimenthoxyethoxysilane, methyldimenthoxypropoxysilane, and phenyltrimenthoxysilane. Of course, mixtures of the above silanes or other derivatives encompassed by formula I can also be used in the medicaments.

Methods of preparing the menthol derivatives of formula I are known in the art. For example, U.S. Patent No. 3,215,719 describes the synthesis of dimenthoxydiethoxysilane from the reaction of menthol and tetraethyl silicate (tetraethoxysilane) with silicon tetrachloride as catalyst. In general, derivatives of formula I may be prepared by the reaction of menthol with a silicon compound having two or more silicon-bonded chlorine atoms, hydrogen atom, or alkoxy groups. It is usually preferred to prepare the desired derivatives by reacting menthol with a silicon compound having two or more of the alkoxy groups bonded to silicon whereby two or more of the alkoxy groups are replaced by the menthoxy group. Generally, it is preferred to expedite the reaction by the use of elevated temperatures and/or suitable catalysts, such as potassium carbonate, sodium hydroxide, sodium borohydride, and metal organic compounds, such as tetrabutyltitanate.

Medicaments are formed by dispersing or dissolving the silicon derivative of menthol into a nonaqueous, physiologically acceptable carrier medium. The carrier acts to dilute the active menthol derivative to facilitate topical application of appropriate levels to the skin. The carrier must be nonaqueous so that the menthol derivatives are not hydrolyzed prior to use. Generally, it is preferred to use a carrier medium that is hydrophobic in character in order to provide further protection against premature hydrolysis of the menthol derivative. Preferred hydrophobic carriers include hydrocarbon liquids and waxes such as petrolatum and mineral oil and silicone fluids such as cyclomethicone and dimethicone. Mixtures of the different carrier components can also be used.

Medicaments containing from 0.5 to 30 parts by weight of menthol derivatives of formula I dispersed or dissolved in 70 to 100 parts by weight of carrier are generally useful for topical applications to the human body. Such medicaments may be formulated in the form of lotions or thickened ointments to provide relief of minor skin irritations and muscle soreness or fatigue. Menthol derivatives may also be formulated as after shave lotions which are used to sooth skin irritation caused by the shaving process.

These medicaments have the advantage of providing a prolonged effect without a strong initial odor and menthol sensation. While medicaments with higher concentrations of free menthol may be used to lengthen the effective period somewhat they have the disadvantage in that they also produce a very pronounced initial odor and characteristic menthol sensation. It is, however, within the scope of the present invention to include some portion of free menthol in the medicaments along with the menthol derivative to control and adjust the relative strength of the initial versus the delayed effect as desired for particular applications.

The medicaments of this invention may also contain other nonactive components such as thickeners, perfumes, and colorants to improve the aesthetics and ease of application. Other active ingredients such as methyl salicylate, clove oil, eucalyptus oil, camphor, spirits of turpentine, and benzocaine can also be included to augment the analgesic effect provided by the menthol derivative. Typically, about 0.5 to 30 parts by weight of such other active ingredients may be used per 100 parts by weight of carrier.

The following examples are presented to illustrate the invention to those skilled in the art and should not be construed as limiting the invention, which is properly delineated in the appended claims. All proportions by parts or percents are by weight unless otherwise stated.

## Example 1

This example shows the synthesis of menthol derivatives which are useful in the medicament compositions of the present invention.

Methyltrimethoxysilane (45 g, 0.33 mol), menthol (150 g, 0.96 mol), and $NaOCH_3$ (0.14 g as catalyst) were mixed in a flask equipped with a fractionating column and a condenser. The mixture was heated for 8 hours during which time the methanol formed by the reaction was removed. The product was analyzed by gas chromatography (GC). The following products and corresponding GC peak areas were detected: methanol (0.4%), methyltrimethoxysilane (0.6%), menthol (29.9%), methylmenthoxydimethoxysilane (10.1%), methyldimenthoxymethoxysilane (44.7%), methyltrimenthoxysilane (5.1%), and high boiler (9%, believed to be a hydrolysis and condensation product resulting from the inadvertent presence of trace amounts of water). The components of the product having two or more menthoxy groups per silicon atom, methyldimenthoxymethoxysilane and methyltrimenthoxysilane, may be separated and used to prepare medicaments compositions of this invention. Alternatively, the unseparated product, in sufficient quantities to provide the required amount of methyldimenthoxymethoxysilane and methyltrimenthoxysilane, may be used to prepare medicament compositions of this invention.

## Example 2

This example shows another synthesis of menthol derivatives using tetraethoxysilane.

Tetraethoxysilane (167 g, 0.80 mol), menthol (250 g, 1.60 mol), and sodium borohydride (0.5 g, as catalyst) were mixed in a flask equipped with a fractionating column and a condenser. The mixture was heated for 8 hours during which time the methanol formed by the reaction was removed. The product was analyzed by gas chromatography (GC). The following products and corresponding GC peak areas were detected: tetraethoxysilane (0.19%), menthol (1.23%), menthoxytriethoxysilane (15.9%), dimenthoxydiethoxysilane (44.5%), trimenthoxyethoxysilane (36.1%), and tetramenthoxysilane (0.5%).

## Example 3

This example shows the preparation of a medicament composition of this invention and compares its long term analgesic effect with a medicament composition containing only free menthol.

Medicament I was prepared by mixing, until homogeneous, 10 g of white petrolatum and 0.2 g of the unseparated product of Example 1. The medicament contained about 1 part of menthol derivatives according to the present invention per 100 parts of the petrolatum carrier. The medicament also contained about 0.3 part of free menthol per 100 parts of carrier.

Comparison medicament II was prepared by melting 0.2 g of menthol and mixing it with 10 g of white petrolatum. This medicament contained 2 parts of free menthol per 100 parts of carrier. A portion of medicament II was applied to the skin of the left arm of a human test subject and an equal portion of medicament I was applied to the skin of the right arm. For the first 15 to 20 minutes after application a cooling sensation, characteristic of menthol, was reported on both arms by the test subject. After about 1 hour, the cooling sensation was no longer detected on either arm. The test subject then performed several minutes of arm exercises. After the exercise, the test subject reported that the cooling sensation had returned to the right arm but not to the left arm. About 2 hours after application, the test subject again reported that the cooling sensation could not be detected on either arm. Again after several minutes of arm exercise, the test subject reported again feeling the cooling sensation on the right arm. This result shows that the medicament with menthol derivative has a longer lasting analgesic effect which may be stimulated by exercise and/or perspiration.

In a second comparison, a small portion of medicament I was placed on the skin of the right nostril of a human test subject. Similarly, an equal portion of comparison medicament II was placed on the skin of the left nostril. A cooling sensation was reported by the test subject for both nostrils immediately after application. After 15 minutes, the cooling sensation was reported to be noticeably greater at the right nostril. After about 45 minutes, the subject reported the absence of any further cooling sensation on the left nostril, but a continuing sensation of cooling at the right nostril. The cooling sensation at the right nostril continued until the test was terminated after 1 hour by washing the treated area. This result shows that the medicament with menthol derivative again has a longer lasting analgesic effect.

## Example 4

This example shows the preparation of a medicament using silicone fluids as the carrier. Further, the long term analgesic effect of a medicament containing menthol derivatives and a medicament containing only free menthol are compared.

Medicament III was prepared by mixing, until homogeneous, 38 g cyclomethicone (decamethylcyclopentasiloxane), 2 g of dimethicone (350 cs), and 10 g of the unseparated product of Example 2. The medicament contained about 20 parts of menthol derivatives according to the present invention per 100 parts of the silicone fluid carrier. The medicament also contained about 0.3 part of free menthol per 100 parts of carrier.

Comparison medicament IV was prepared by melting 10 g of menthol and mixing it with 40 g of the same mixture of silicones used in medicament III. Comparison medicament IV contained 25 parts free menthol per 100 parts of carrier. A portion of comparison medicament IV was applied to the skin of the left arm of a human test subject and an equal portion of medicament III was applied to the skin of the right arm. The subject reported a cooling sensation on both arms for about 2 hours after the application. After the cooling sensation had disappeared from both arms, the treated area on each arm was rubbed vigorously by hand for a few seconds. After the rubbing, the subject reported a return of the cooling sensation to the right arm, but not to the left arm.

In a second comparison, a portion of medicament III was applied to the skin on the left side of the torso of a human test subject and an equal portion of comparison medicament IV was applied to the skin on the right side of the torso. Initially after application, the subject reported a strong cooling sensation on the right side and a mild cooling sensation on the left side. After 1 hour, a mild cooling sensation was reported on each side of approximately equal intensity. After 4 hours, a slight cooling sensation on the left side was still detected by the subject, but no effect could be detected on the right side. After 6 hours, the subject exercised for several minutes and began perspiring. The subject reported a strong cooling sensation once again at the treated area on the left side of the torso. These results show that the medicament with menthol derivative again has a longer lasting analgesic effect which may be stimulated by exercise and/or perspiration.

## Claims

1. A medicament comprising (a) from 0.5 to 30 parts by weight of menthol derivative represented by the formula

$$R_{4-x}Si(OC_{10}H_{19})_x$$

wherein the group $OC_{10}H_{19}$ represents the menthoxy radical; R denotes a radical having 1 to 6 carbon atoms selected from the group consisting of alkyl radicals, alkoxy radicals, the phenyl radical, and the phenoxy radical; and x has the value of 2, 3, or 4 and (b) 70 to 100 parts by weight of a nonaqueous, physiologically acceptable carrier.

2. The medicament according to claim 1 wherein the carrier is selected from the group consisting of petrolatum, mineral oil, cyclomethicone, and dimethicone.

3. The medicament according to claim 2 wherein R denotes a methyl, ethyl, ethoxy, or propoxy radical.

4. The medicament according to claim 3 wherein the carrier is petrolatum.

5. The medicament according to claim 3 wherein the carrier is cyclomethicone.

6. A method of treating skin to elicit an analgesic or antipruritic response comprising contacting the skin with medicament defined in claim 1.